# EUROPÄISCHE PATENTSCHRIFT

(11) **EP 1 899 287 B1**
(45) Veröffentlichungstag und Bekanntmachung des Hinweises auf die Patenterteilung: **18.09.2013**
(21) Anmeldenummer: 06776083.5
(22) Anmeldetag: 27.06.2006
(51) Int. Cl.: B01J 19/24, C07C 17/02, C07C 19/045, F28D 5/00

(54) **VERFAHREN UND VORRICHTUNG ZUR NUTZUNG DER BEI DER HERSTELLUNG VON 1,2-DICHLORETHAN ANFALLENDEN REAKTIONSWÄRME**
METHOD AND DEVICE FOR USING REACTION HEAT DURING THE PRODUCTION OF 1,2-DICHLORETHANE
PROCEDE ET DISPOSITIF POUR UTILISER LA CHALEUR DEVELOPPEE PAR LA REACTION LORS DE LA PRODUCTION DE 1,2-DICHLORETHANE

(30) Priorität: 28.06.2005 DE 102005030512; 28.06.2005 DE 102005030511; 15.09.2005 DE 102005044177
(43) Veröffentlichungstag der Anmeldung: 19.03.2008
(73) Patentinhaber: ThyssenKrupp Uhde GmbH, 44141 Dortmund (DE); Vinnolit GmbH & Co. KG, 84504 Burgkirchen (DE)
(72) Erfinder: PETERSEN, Sven, 65779 Kelkheim (DE); BENJE, Michael, 64289 Darmstadt (DE); KAMMERHOFER, Peter, 84508 Burgkirchen (DE)
(86) Internationale Anmeldenummer: PCT/EP2006/006163
(87) Internationale Veröffentlichungsnummer: WO 2007/000304

(56) Entgegenhaltungen:
- EP-A- 1 393 798
- WO-A2-01/34542
- DE-A1- 4 039 960

## Beschreibung

Die Erfindung richtet sich auf ein Verfahren und eine Vorrichtung zur Nutzung der bei der Herstellung von 1,2-Dichlorethan, im folgenden als EDC bezeichnet, anfallenden Reaktionswärme. EDC dient überwiegend als Zwischenprodukt bei der Herstellung von monomerem Vinylchlorid, im folgenden als VCM bezeichnet, woraus letztlich Polyvinylchlorid, PVC, hergestellt wird. Bei der Umsetzung von EDC zu VCM entsteht Chlorwasserstoff HCl. EDC wird daher bevorzugt aus Ethen C₂H₄ und Chlor Cl₂ derart hergestellt, dass hinsichtlich des bei den Umsetzungen erzeugten und verbrauchten Chlorwasserstoffes HCl eine ausgewogene Bilanz entsprechend den folgenden Reaktionsgleichungen erreicht wird:

Cl₂ + C₂H₄ → C₂H₄Cl₂ (Rein-EDC) + 218 kJ/Mol (1)

C₂H₄Cl₂ (Spalt-EDC) → C₂H₃Cl (VCM) + HCl - 71 kJ/Mol (2)

C₂H₄ + 2 HCl + ½ O₂ → C₂H₄Cl₂ (Roh-EDC) + H₂O + 238 kJ/Mol (3)

Das Verfahren zur Herstellung von VCM mit ausgewogener HCl-Bilanz, im folgenden kurz "ausgewogenes VCM-Verfahren" genannt, besitzt:
- eine Direktchlorierung, in der aus Ethen C₂H₄ und Chlor Cl₂ der eine Teil des benötigten EDC erzeugt wird und als sogenanntes Rein-EDC abgegeben wird; die Verwertung der bei dieser Direktchlorierung erzeugten Reaktionswärme ist zentraler Bestandteil der Erfindung;
- eine Oxichlorierung, in der aus Ethen C₂H₄, Chlorwasserstoff HCl und Sauerstoff O₂ der andere Teil des EDC erzeugt wird und als sogenanntes Roh-EDC abgegeben wird;
- eine fraktionierende EDC-Reinigung, in der das Roh-EDC zusammen mit dem aus der VCM-Fraktionierung rezirkulierten Rück-EDC von den in der Oxichlorierung und von den in der EDC-Pyrolyse gebildeten Nebenprodukten befreit wird, um ein für den Einsatz in der EDC-Pyrolyse geeignetes, sogenanntes Feed-EDC zu gewinnen; die Nutzung der Reaktionsabwärme der Direktchlorierung in der EDC-Reinigung ist zentraler Bestandteil der Erfindung;
- eine EDC-Pyrolyse, in der das Rein-EDC mit dem Feed-EDC zusammengeführt und in der das dann Spalt-EDC genannte Gemisch thermisch gespalten wird; das erhaltene Spaltgas enthält VCM, Chlorwasserstoff HCl und nichtumgesetztes EDC sowie Nebenprodukte;
- eine VCM-Fraktionierung, in der das als Produkt gewünschte Rein-VCM aus dem Spaltgas abgetrennt und die anderen wesentlichen Spaltgasbestandteile Chlorwasserstoff HCl und nichtumgesetztes EDC als Wertstoffe gesondert zurückgewonnen und als wiederverwertbarer Einsatz als Rück-HCl bzw. Rück-EDC im ausgewogenen VCM-Verfahren rezirkuliert werden.

Anlagen, die der reinen EDC-Herstellung dienen und nicht nach dem ausgewogenen VCM-Verfahren arbeiten, sind meist als reine Direktchlorierungen ausgeführt, die nur nach Gleichung (1) verfahren. Die Erfindung richtet sich gleichermaßen auf Anlagen, die nach dem ausgewogenen VCM-Verfahren ausgelegt sind, als auch auf solche, die nur über eine Direktchlorierung verfügen, sowie auf Mischformen der beiden Typen.

Das in der Direktchlorierung benötigte Chlor Cl₂ wird üblicherweise in einer Anlage zur Elektrolyse aus Natriumchlorid NaCl erzeugt. Als Koppelprodukt entsteht hierbei Natronlauge NaOH mit einer Konzentration von ca. 33 %. Wegen der hohen Giftigkeit des erzeugten Chlors Cl₂ ist man bestrebt, einen weiten Transport möglichst zu vermeiden. Meist befindet sich daher in unmittelbarer Nähe einer Anlage zur Herstellung von Natronlauge NaOH und Chlor Cl₂ eine Anlage zur Direktchlorierung von Ethylen C₂H₄, in der das Chlor Cl₂ unmittelbar weiter verarbeitet wird.

Die Anlage zur Direktchlorierung von Ethylen muss sich dabei nicht in einem Anlagenverbund befinden, sondern kann im so genannten "Stand-alone-Betrieb" EDC produzieren, das als relativ ungefährliche "Transportform" des Chlors zu anderen Anlagenstandorten transportiert wird, um dort zu VCM verarbeitet zu werden. Insbesondere das nach dem z.B. in der WO 01/34542 A2 beschriebenen Verfahren hergestellte EDC ist so rein, dass es keiner weiteren destillativen Aufarbeitung bedarf. Daher entfällt, wenn eine solche Anlage im "Stand-alone-Modus" betrieben wird, die Möglichkeit der Wärmerückgewinnung durch Kolonnenbeheizung von EDC-Destillationskolonnen, etwa solcher, die im Verbund des "ausgewogenen VCM-Verfahrens", welches sich aus Direktchlorierung, Oxychlorierung und EDC-Spaltung zusammensetzt, vorhanden wären.

Daher muss bei dieser Konstellation die erhebliche Reaktionswärme durch große Mengen an Kühlwasser und/oder durch Luftkühler abgeführt werden, was aber beides aus wirtschaftlichen Gründen nicht wünschenswert ist. Eine Aufgabe der Erfindung ist es daher, die Abwärme der Direktchlorierung einer Nutzung zuzuführen und den Bedarf an Kühlwasser deutlich zu verringern.

Sofern die Direktchlorierung innerhalb eines ausgewogenen VCM-Verfahrens eingesetzt wird, muss berücksichtigt werden, dass die in der EDC-Pyrolyse im Spaltgas entstehenden Begleitstoffe die Produkt-Reinheit des VCM herabsetzen. Die Reinigung des VCM durch Entfernung der Begleitstoffe ist dementsprechend aufwendig. Daher wird ein weitestgehend von Verunreinigungen befreites Spalt-EDC in der EDC-Pyrolyse eingesetzt. Aus der großen Zahl von Techniken, wie die entsprechenden und nachteiligen Nebenprodukte und/oder Begleitstoffe vermieden bzw. gegebenenfalls abgereinigt werden können, sei wieder auf die Schrift WO 01/34542 A2 verwiesen, insbesondere auf den dort gewürdigten Stand der Technik. Hierbei konnte gezeigt werden, dass die Reaktionswärme, die beim Direktchlorierungsverfahren durch Umsetzung von Ethen C₂H₄ und Chlor Cl₂ in flüssigem EDC frei wird, ausreicht, um die Reinigungskolonnen für erzeugtes EDC im ausgewogenen VCM-Verfahren zu betreiben.

Nachteilig ist bei dem dort vorgestellten Verfahren jedoch, dass die Abwärmenutzung des EDC nur bei relativ hohem Temperaturniveau, d.h. überwiegend oberhalb von ca. 100 °C, stattfinden kann. Obwohl der Betrieb der Vorrichtungen zur Reinigung des EDC allein aus Abwärme erreicht werden konnte, muss die weitere Abkühlung des erzeugten EDC, z.B. für die spätere Verwendung, immer noch mittels Kühlwasser durchgeführt werden, wobei immer noch große Mengen Kühlwasser erforderlich sind.

Weiter nachteilig ist bei dem dort vorgestellten Verfahren auch, dass die zur Beheizung der Reinigungskolonnen eingesetzte Reaktionswärme die Abführung einer korrespondierenden Wärmemenge zur Kondensation der Brüden erfordert. Diese Abführung geschieht nach dem herkömmlichen Stand der Technik üblicherweise ebenfalls mittels Kühlwasser, welches in großer Menge bereitgestellt werden muss.

Eine weitere Aufgabe der Erfindung ist es daher, die Abwärmenutzung des ausgewogenen VCM-Verfahrens, insbesondere der Direktchlorierung weiter zu optimieren und den gesamten Bedarf an Kühlwasser deutlich zu verringern.

Die Erfindung löst die Aufgabe dadurch, dass die Reaktionswärme der Bildung von 1,2-Dichlorethan im Direktchlorierungsreaktor zumindest teilweise für die Eindampfung von NaOH, welches bei der NaCl-Elektrolyse bei der Herstellung des für die Direktchlorierung benötigten Chlors als Koppelprodukt erzeugt wird, genutzt wird.

Insbesondere in entlegenen Gebieten spielen die Transportkosten für den Abtransport der bei der NaCl-Elektrolyse erzeugten Natronlauge NaOH eine wichtige Rolle. Diese Transportkosten können deutlich gesenkt werden, wenn die bei einer Konzentration von ca. 33 % erzeugte Lauge auf 50 % eingedampft wird. Eine derartige Anlage zur Eindampfung von Natronlauge NaOH kann z.B. unter Vakuum bei einem Absolutdruck von 133 mbar und einer Temperatur von 60 °C betrieben werden. Die Eindampfung kann natürlich auch von anderen Konzentrationen als 33 % auf andere Konzentrationen als auf 50 % erfolgen, je nach Abnehmerwunsch und Abwärmeanfall.

Die Reaktionswärme, die bei der Direktchlorierung frei wird, kann auf verschiedene Arten für die Natronlaugeeindampfung eingesetzt werden. Die folgenden Ausgestaltungen des Verfahrens sind gut kombinierbar und ermöglichen eine große Flexibilität bei der Anpassung der Wärmenutzung an vorhandene Anlagen sowie bei der Neukonzeption.

In einer Ausgestaltung der Erfindung wird die Kondensationswärme des aus der Direktchlorierung abgezogenen EDC-Dampfes zumindest teilweise für die Eindampfung der erzeugten Natronlauge eingesetzt. Eine derartige Ausgestaltung bietet sich vor allem dann an, wenn eine Direktchlorierung im "Stand-alone-Betrieb" eingesetzt wird, oder wenn die destillative Reinigung des im ausgewogenen VCM-Verfahren erzeugten EDC nicht die gesamte auf diese Weise zur Verfügung gestellte Reaktionswärme aufnehmen kann. Hierbei werden EDC-Brüden vom Kopf des Direktchlorierungsreaktors benutzt, um mit deren Kondensationswärme mantelseitig Verdampferrohre zu beheizen, die als Fallfilm-Verdampferrohre ausgebildet sein können und in denen Natronlauge eingedampft wird. Das Kondensat aus Rein-EDC kann auch noch in einem weiteren Wärmetauscher, beispielsweise einem Einsteckröhrentauscher, unter weiterer Abkühlung zur Eindampfung der Natronlauge dienen.

In einer weiteren Ausgestaltung der Erfindung wird die fühlbare Wärme eines aus dem Reaktor abgezogenen Flüssig-EDC-Kreislaufstroms ebenfalls dazu benutzt, Natronlauge einzudampfen. Bei der Kombination mit anderen Reaktionswärme abführenden Strömen ist darauf zu achten, dass Katalysator-haltiges EDC, welches in den Reaktorumlauf des Direktchlorierungsreaktors zurückgeführt werden soll, nicht mit Rein-EDC, welches als Produkt aus dem Prozess geführt werden soll, vermischt wird.

In einer weiteren Ausgestaltung der Erfindung wird das erzeugte EDC, welches aus dem Reaktor der Direktchlorierung dampfförmig oder flüssig abgezogen wird, zunächst zur indirekten Beheizung von Reinigungskolonnen genutzt, und erst nachdem das EDC einen Teil seiner Wärmeenergie auf relativ hohem Temperaturniveau dort abgegeben hat, zur weiteren Energieabgabe in die Natronlaugeeindampfung weitergegeben, wo es Wärmeenergie auf geringerem Temperaturniveau im indirekten Wärmetausch an Natronlauge abgibt. Sofern ein ausgewogenes VCM-Verfahren zur Herstellung von EDC und VCM eingesetzt wird, können bevorzugt die hierfür erforderlichen Reinigungskolonnen beheizt werden, bevor die restliche Reaktionswärme zur Eindampfung der Natronlauge genutzt wird.

Weitere Ausgestaltungen der Erfindung verwenden den Umstand, dass dieselbe Reaktionswärme, die der indirekten Beheizung von Reinigungskolonnen zugeführt wird, bei der anschließenden Kondensation der Brüden ebendieser Reinigungskolonnen wieder abgeführt werden muss, die Reinigungskolonne also sozusagen nur durchläuft und dabei thermodynamisch entwertet wird. Ein beträchtlicher Teil dieser durchlaufenden Reaktionswärme kann aber trotz abgesenktem Temperaturniveau ebenfalls noch für die Natronlaugeeindampfung verwendet werden.

So werden in einer weiteren Ausgestaltung der Erfindung die EDCenthaltenden Brüden einer Destillationskolonne zur Entfernung von höher als EDC siedenden Komponenten für die Eindampfung von Natronlauge verwendet. Eine solche Destillationskolonne ist als sogenannte Hochsiederkolonne üblicherweise Bestandteil des ausgewogenen VCM-Verfahrens. Sie ist nicht zu verwechseln mit der sogenannten Vakuumkolonne, die sich an die Hochsiederkolonne anschließt, und deren Kopftemperatur für den erfindungsgemäßen Einsatz nicht ausreichend hoch wäre.

Ebenfalls werden in einer weiteren Ausgestaltung der Erfindung die wasserhaltigen Brüden einer Destillationskolonne zur Entfernung von Wasser und leichter als 1,2-Dichlorethan siedenden Komponenten für die Eindampfung von Natronlauge verwendet. Eine solche Destillationskolonne ist als sogenannte Leichtsiederkolonne üblicherweise Bestandteil des ausgewogenen VCM-Verfahrens.

Es sind auch Verfahren bekannt, bei denen das in der Direktchlorierung erzeugte EDC dampfförmig abgezogen und von dort aus direkt in eine Destillation geleitet wird, eine Direktchlorierung dieser Art wird beispielsweise in der US 4,873,384 beschrieben. Der Direktchlorierungsreaktor bildet hierbei gleichzeitig auch den Sumpfaufkocher der nachfolgenden Reinigungskolonne oder integriert diesen in den Sumpf der Reinigungskolonne selbst. Die Reaktionswärme durchläuft auf diese Weise die angeschlossene Destillationskolonne und fällt bei der Brüdenkondensation zur Abführung an. In einer Ausgestaltung der Erfindung wird die Kondensationswärme der Brüden, die bei der destillativen Reinigung von in einem Direktchlorierungsreaktor aus Ethen und Chlor erzeugtem EDC anfallen, zumindest teilweise für die Eindampfung der erzeugten Natronlauge eingesetzt.

Weitere Ausgestaltungen der Erfindung betreffen die zum Einsatz kommenden apparativen Einrichtungen zur Übertragung der Wärmeenergie des EDC an die einzudampfende Natronlauge NaOH. Hierbei kommt hauptsächlich ein stehender Rohrbündelwärmetauscher, vorzugsweise ein Fallfilmverdampfer, mit 2 festen Rohrplatten und einem NaOH-Sumpfteil zum Einsatz, bei dem die Natronlauge NaOH rohrinnenseitig von oben nach unten und der Wärmeträger, also entweder EDC oder Brüden von Destillationskolonnen, auf der Außenseite der Rohre geführt wird.

Sofern dampfförmiges EDC oder Brüden aus Destillationskolonnen in der Natronlaugeeindampfung zum Einsatz kommt, findet die Wärmeübertragung im Rohrbündel im Gleichstrom statt. Der oben auf das Rohrbündel aufgegebene EDC-Dampf bzw. EDC-Brüden kondensiert dabei und kann unten flüssig abgezogen werden.

Sofern flüssiges EDC in der Natronlaugeeindampfung zum Einsatz kommt, kann die Wärmeübertragung sowohl im Rohrbündel, dann aber zweckmäßiger Weise im Gegenstrom, als auch mittels eines eingesteckten Wärmetauscherbündels im Natronlaugesumpf, als auch mittels eines außerhalb des Natronlaugesumpfes gelegenen und im Umlauf betriebenen Wärmetauschers, z.B. vom Kettle-Typ, erfolgen.

Alle oben beschriebenen Methoden sind auch additiv bzw. in Kombination anwendbar. Soll das oben gelegene Rohrbündel sowohl mit EDC-Dampf als auch mit flüssigem EDC betrieben werden, kann das Rohrbündel horizontal geteilt werden. Selbstverständlich ist darauf zu achten, dass die einzelnen Brüdenströme verschiedener Destillationskolonnen nicht miteinander vermischt werden dürfen.

Üblicherweise wird die Natronlaugeverdampfung in mehrstufigen Verdampfungsanlagen durchgeführt, die beispielsweise aus mehreren hintereinander geschalteten Verdampfungsapparaten bestehen. Daher können die hier beschriebenen erfindungsgemäßen Maßnahmen auch getrennt an verschiedenen Stufen bzw. Verdampfungsapparaten einer solchen Anlage eingesetzt werden. So kann beispielsweise eine Stufe mit dampfförmigen EDC beheizt werden, während eine andere Stufe mit flüssigem EDC beheizt wird. Die erfindungsgemäßen Maßnahmen können aber auch lediglich an einer Stufe oder an mehreren Stufen gleichzeitig einer mehrstufigen Anlage zur Eindampfung von Natronlauge eingesetzt werden. Auch ist es möglich, verschiedene Stufen mit verschiedenen Vakuumdrücken zu betreiben, um unterschiedliche Temperaturen der einzelnen Wärmeträger zulassen zu können.

Die Erfindung wird nachfolgend anhand von 8 Zeichnungen näher erläutert, wobei das erfindungsgemäße Verfahren aber nicht auf diese speziellen Fälle beschränkt ist:
- Fig. 1 zeigt eine mögliche Verschaltung mit einem Direktchlorierungsreaktor und einer Natronlaugeeindampfung entsprechend den Verfahrensansprüchen 2 und 3,
- Fig. 2 zeigt eine mögliche Verschaltung mit einem Direktchlorierungsreaktor mit angeschlossener Reinigungskolonne und einer Natronlaugeeindampfung entsprechend Verfahrensanspruch 4,
- Fig. 3 und Fig. 4 zeigen jeweils eine mögliche Verschaltung einer Natronlaugeeindampfung mit einem Direktchlorierungsreaktor und einer Wärmerückgewinnung nach dem ausgewogenen VCM-Verfahren, in Anlehnung an die Lehre der WO 01/34542 A2 und entsprechend Verfahrensanspruch 5,
- Fig. 5 zeigt eine mögliche Verschaltung mit einem Direktchlorierungsreaktor, Reinigungskolonnen mit Brüdenkondensation und einer Natronlaugeeindampfung entsprechend den Verfahrensansprüchen 6 und 7,
- Fig. 6 bis 8 zeigen beispielhafte Ausführungsformen der Vorrichtung für die Natronlaugeeindampfung entsprechend den Vorrichtungsansprüchen.

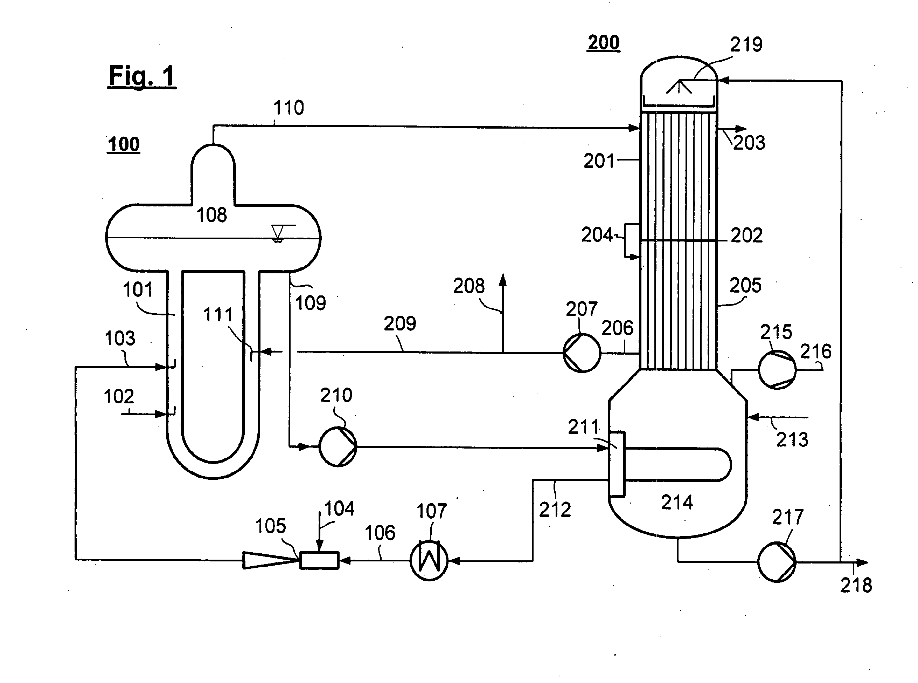

Fig. 1 zeigt eine Verschaltung einer Natronlaugeeindampfung mit einem Direktchlorierungsreaktor, der im "Stand-alone-Betrieb" EDC erzeugt, und dessen Reaktionswärme sowohl entsprechend Verfahrensanspruch 2 mittels dampfförmigem als auch entsprechend Verfahrensanspruch 3 mittels flüssigem EDC die Natronlaugeeindampfung beheizt.

Die Direktchlorierung 100 besteht aus einer flüssigkeitsgefüllten Schlaufe 101, einer Einspeisung von Ethylen 102, einer Zugabe von in EDC gelöstem Chlor 103, wobei das Chlorgas 104 vorher im Injektor 105 in flüssigem EDC 106 gelöst wurde, welches im EDC-Kühler 107 zuvor zur Verbesserung der Löslichkeit auf eine niedrige Temperatur abgekühlt wurde, ferner einem Ausgasgefäß 108, einer Abzugsvorrichtung für flüssiges EDC 109, einer Abzugsvorrichtung für gasförmiges EDC 110 und einer Zuführstelle von Umlauf-EDC 111, wobei die jeweiligen Zuführstellen und Abzugsvorrichtungen aus praktischen Gründen auch mehrfach ausgeführt sein können. In der flüssigkeitsgefüllten Schlaufe 101 reagieren Chlor und Ethylen miteinander zu siedendem EDC, welches im Ausgasgefäß 108 zusammen mit nicht reagierten Ausgangsstoffen und inertem Begleitgas ausdampft.

Gasförmiges EDC 110 wird in den oberen Mantelraum 201 des hier horizontal geteilt dargestellten Rohrbündelwärmetauschers 202 der Natronlaugeeindampfung 200 gegeben, wo es unter Wärmeabgabe kondensiert, dabei jedoch nicht wesentlich unterkühlt wird, um Druckschwankungen des EDC-Dampfes zu vermeiden. Nicht kondensierbare Bestandteile werden über den Inertgasabzug 203 abgeführt. Hierbei ist durch geeignete technische Maßnahmen sicher zu stellen, dass sich im Mantelraum des Rohrbündelwärmetauschers kein explosionsfähiges Gasgemisch bilden kann. Solche Maßnahmen sind dem Fachmann bekannt und nicht Gegenstand der Erfindung. Das EDC-Kondensat 204 wird in den unteren Mantelraum 205 des horizontal geteilten Rohrbündelwärmetauschers 202 abgeleitet, wo das flüssige EDC abgekühlt wird. Die Ableitung des kondensierten EDC in den unteren Mantelraum kann optional durch eine Pumpe (nicht dargestellt) unterstützt werden.

Das gekühlte Rein-EDC 206 wird von der Kondensat-Pumpe 207 aus dem horizontal geteilten Rohrbündelwärmetauscher 202 abgezogen und in zwei Teilströme aufgeteilt: Produkt-EDC 208 und Umlauf-EDC 209. Das Produkt-EDC 208 wird nach Abkühlung in einem Produktkühler (nicht dargestellt) zur Anlagengrenze gefahren, das Umlauf-EDC 209 wird zurück in den Reaktor gefahren.

Das aus der Abzugsvorrichtung für flüssiges EDC 109 abgezogene, Katalysator-haltige EDC wird von der EDC-Pumpe 210 in den Einsteckkühler 211, der im Sumpfteil 214 der Natronlaugeeindampfung 200 angebracht ist, gefördert und wird dort abgekühlt. Das gekühlte EDC 212 aus dem Einsteckkühler 211 wird in dem Kreislaufkühler 107 weiter abgekühlt und der Injektordüse 105 zugeführt, wo es als Treibstrom das Chlor 104 ansaugt und auflöst. Der Strom 103 von in EDC gelöstem Chlor wird dann dem Direktchlorierungsreaktor 100 zugeführt.

33-%ige Natronlauge 213 wird in den Sumpfteil 214 der Natronlaugeeindampfung 200 eingegeben und unter Vakuum eingedampft. Die Druckhaltung erfolgt durch die Vakuumpumpe 215, die den freiwerdenden Wasserdampf 216 abführt. Die Natronlaugepumpe 217 führt einen Teil der auf ca. 50 % konzentrierten Natronlauge als Produkt-NaOH 218 ab und fördert einen anderen Teil zum Natronlauge-Verteiler 219, der die einzudickende Natronlauge in das Rohrinnere des Rohrbündelwärmetauschers 202 aufteilt. Die Verdampfungsenergie für die Eindampfung wird hierbei durch die Kondensationswärme und/oder die fühlbare Wärme des kondensierten EDC aufgebracht.

Zur Veranschaulichung dient das folgende Zahlenbeispiel auf der Basis einer Simulationsrechnung, wobei eine Anlage mit einer Kapazität von 250,000 Jahrestonnen EDC zugrunde gelegt wird. Bei einer Anlage dieser Größe beträgt die Reaktionsenthalpie ca. 19,1 MW (218 kJ/mol EDC). Einer Kapazität von 250,000 Jahrestonnen EDC entspricht eine Chlormenge von 22,5 t Chlor/h, was wiederum einer Natronlaugeproduktion von ca. 25,4 t/h (gerechnet als 100 % NaOH) entspricht. Die Natronlauge fällt mit einer Konzentration von 33 % bei einer Temperatur von ca. 80°C an und wird durch Vakuumeindampfung auf 50 % aufkonzentriert. Dies entspricht einer zu verdampfenden Wassermenge von ca. 26,2 t/h bzw. einer Wärmeleistung von 14,6 MW.

Dieser Wärmebedarf kann durch die Abwärme der Direktchlorierung komplett gedeckt werden; somit sind bei dieser Anwendung ca. 76 % der Reaktionswärme rückgewinnbar, was ein Vorteil der Erfindung ist. Die Verdampfung wird bei Unterdruck von ca. 133 mbar absolut und einer Temperatur von 60°C betrieben. Die restliche abzuführende Reaktionswärme wird durch Wärmetauscher in der Direktchlorierungsanlage abgeführt.

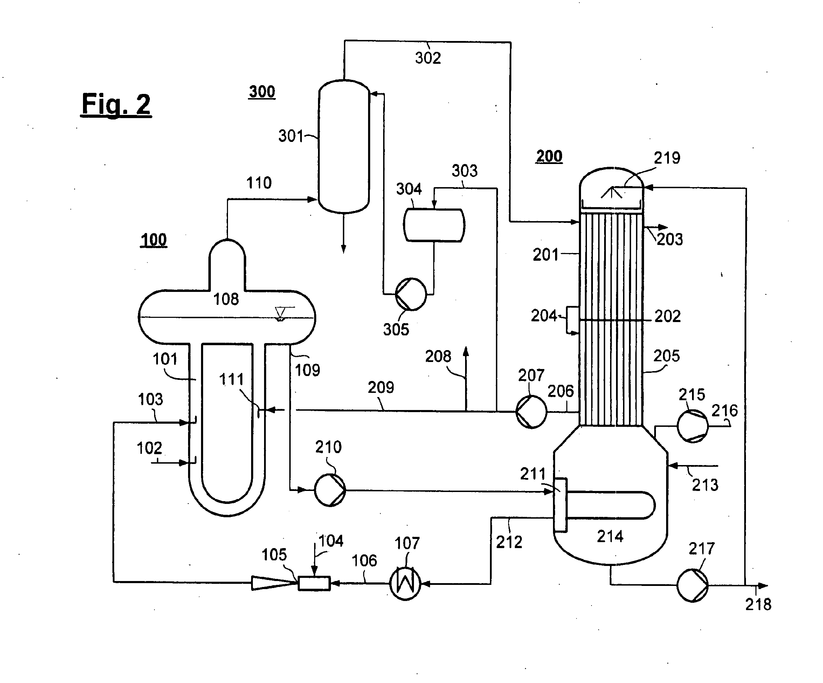

Fig. 2 zeigt einen Direktchlorierungsreaktor mit angeschlossener Destillation 300 zur Reinigung des erzeugten EDC. Der Aufbau der Baugruppen Direktchlorierung 100 und Natronlaugeeindampfung 200 ist dabei der gleiche wie in Fig. 1 beschrieben. Im Unterschied zu der in Fig. 1 beschriebenen Verfahrensweise wird der EDC-Brüden, der dem Direktchlorierungsreaktor entnommen wird, zunächst destillativ in der Reinigungskolonne 301 gereinigt. Der Brüden 302 der Reinigungskolonne 301 wird in den oberen Mantelraum 201 der Natronlaugeeindampfung 200 aufgegeben. Die Natronlaugeeindampfung 200 dient hierbei als Brüdenkondensator der Reinigungskolonne 301. Das Brüdenkondensat 303 wird von der Kondensat-Pumpe 207 in die Kopfvorlage 304 der Reinigungskolonne 301 gefördert, und von dort aus mittels der Vorlagepumpe 305 auf den Kopf der Reinigungskolonne 301 gegeben.

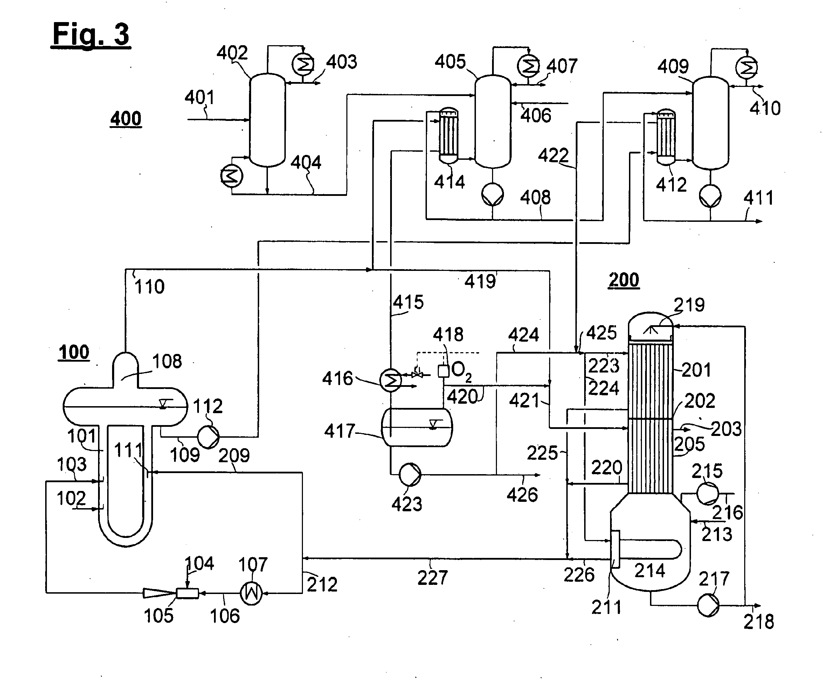

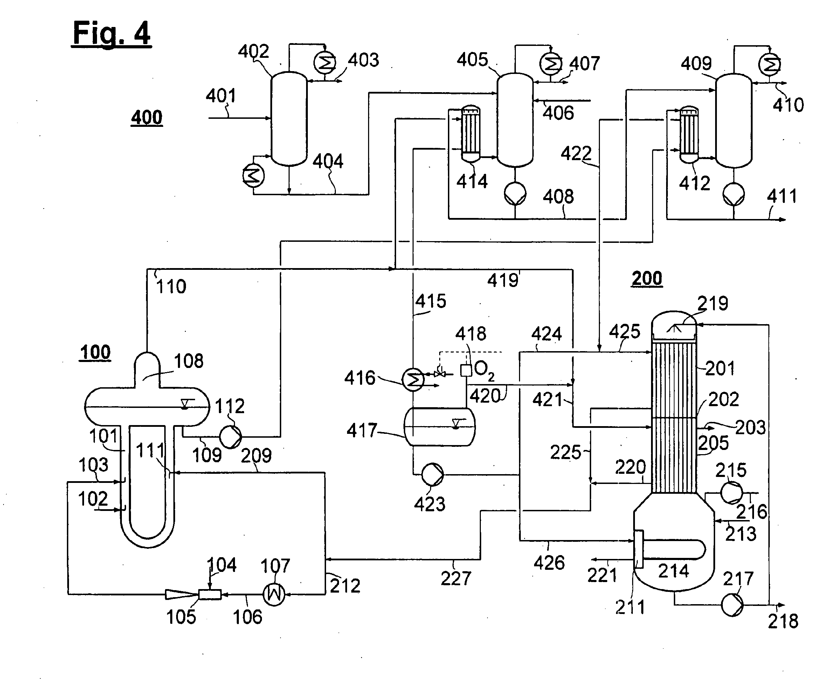

Fig. 3 und Fig. 4 zeigen, wie im ausgewogenen VCM-Verfahren in der EDC-Reinigung 400 das EDC aus der Oxichlorierung und das nicht umgesetzte EDC aus der EDC-Pyrolyse in einer energieaufwändigen EDC-Destillation gereinigt werden. Das Roh-EDC 401 aus der Oxichlorierung, die hier nicht dargestellt ist, wird in einer Leichtsiederkolonne 402 zunächst von Wasser und Leichtsiedern getrennt, die über die Leichtsiederleitung 403 abgeführt werden. Danach wird das als Sumpfprodukt der Leichtsiederkolonne erhaltene EDC, welches noch Hochsieder enthält, über die EDC-Leitung 404 der Hochsiederkolonne 405 zugeführt. Das nicht umgesetzte EDC aus der EDC-Pyrolyse enthält ebenfalls Hochsieder und wird über die EDC-Leitung 406 der Hochsiederkolonne 405 zugeführt.

In der Hochsiederkolonne 405 werden die zugeführten Stoffströme fraktioniert. Gereinigtes EDC wird am Kopf der Hochsiederkolonne 405 über die Brüdenleitung 407 abgezogen und als reines EDC gewonnen. Im Sumpf der Hochsiederkolonne 405 reichem sich die Hochsieder an. Der Sumpfstrom 408 der Hochsiederkolonne 405 wird in der Vakuumkolonne 409 aufgearbeitet. Am Kopf der Vakuumkolonne 409 wird reines EDC über die EDC-Brüdenleitung 410 abgezogen. Der Sumpfabzug 411 der Vakuumkolonne 409 besteht aus Hochsiedern und einem kleinen Restanteil EDC.

Die Beheizung der Kolonnen 405 und 409 erfolgt dabei folgendermaßen: Aus dem Direktchlorierungsreaktor 100 wird flüssiges EDC 109 abgezogen und an den Fallfilmverdampfer 412 der Vakuumkolonne 409 als Heizmedium zu- und abgeführt. vom gasförmigen EDC 110 der Direktchlorierung 100 wird EDC-Brüden 413 abgezweigt und dem Fallfilmverdampfer 414 der Hochsiederkolonne 405 als Heizmedium zugeführt. In den Fallfilmverdampfern 412 und 414 fließt die zu erhitzende Flüssigkeit vom Kopf des Verdampferkörpers als gleichmäßig verteilter, siedender Film auf Grund der Schwerkraft auf der Innenseite der Heizrohre herab und verdampft dabei teilweise. Auf der Außenseite des Fallfilmverdampfers 414 kondensiert der größte Teil des EDC-Brüden. Selbstverständlich können auch andere Wärmetauscher, z.B. normale Thermosiphon-Reboiler, verwendet werden.

Der Austrittstrom 415 des Fallfilmverdampfers 414 kann einem optionalen Trimmkondensator 416 zugeführt werden, der zur Regelung des Systems dient. Flüssiges EDC wird im Anschluss daran in der Vorlage 417 von nichtkondensierbaren Anteilen getrennt. Hierbei muss durch geeignete Maßnahmen sichergestellt werden, dass sich während der Kondensation kein explosives Gemisch zwischen Sauerstoff, restlichem Ethen und EDC-Dampf bilden kann. Daher misst beispielsweise ein Sauerstoffmessgerät 418 den Sauerstoffgehalt und eine damit verbundene Regelungseinrichtung regelt die Zulaufmenge an Kühlmedium des Trimmkondensators 416 entsprechend, es können aber auch noch weitere Regelungseinrichtungen auf den Trimmkondensator 416 aufgeschaltet werden. Falls eine Regelmöglichkeit nicht erforderlich ist, kann der Trimmkondensator 416 auch entfallen. Die Bildung eines explosionsfähigen Gasgemischs kann auch durch andere Maßnahmen verhindert werden, die nicht Gegenstand der Erfindung sind.

Dem jeweiligen Bedarf der Hochsiederkolonne 405 entsprechend kann es auch sein, dass zumindest zeitweise ein Überangebot an EDC-Dampf vorliegt. In diesem Fall wird ein EDC-Teilstrom 419 vom gasförmigen EDC 110 abgezweigt und mit den nichtkondensierbaren Anteilen 420 aus der Vorlage 417 zusammengeführt. Gemeinsam dient dieser EDC-Dampfstrom 421 der Beheizung des unteren Abschnitts 205 des Rohrbündels der Natronlaugeeindampfung 200, wobei das EDC kondensiert und als EDC-Kondensat 220 abgezogen wird. Die nicht kondensierbaren Anteile werden als Abgas 221 abgezogen und einer weiteren, hier nicht dargestellten Behandlung unterzogen.

Aus dem Ausdampfbehälter 108 wird mit der Kreislaufpumpe 112 ein flüssiger EDC-Strom 109 abgezogen und dem Fallfilmverdampfer 412 zur Beheizung der Vakuumkolonne 409 zugeführt. Der nach Abgabe fühlbarer Wärme leicht abgekühlte EDC-Strom 422 wird mit einem Teil des aus der Vorlage 417 mittels der Pumpe 423 abgezogenen Rein-EDC 424 zum Rein-EDC 425 verbunden und der Natronlaugeeindampfung 200 zugeführt. Der andere Teil des aus der Vorlage 417 mittels der Pumpe 423 abgezogenen Rein-EDC 424 dient als Produkt-EDC 426.

Fig. 3 zeigt hierbei einen Weg, Produkt-EDC bei höherer Temperatur zu gewinnen. Diesen Weg wird der Fachmann wählen, wenn er das produzierte EDC unmittelbar im nächsten Prozessschritt zu VCM weiterverarbeiten will, weil er sich dann einen Teil der Wiederaufheizung sparen kann. Hierbei wird Produkt-EDC 426 ohne weitere Abwärmenutzung verwendet. Möchte man EDC aber in große, drucklose Vorratstanks fördern, kann der in Fig. 4 aufgezeigte Weg genutzt werden: Dort wird das Produkt-EDC 426 im Einsteckkühler 211, der im Sumpfteil 214 der Natronlaugeeindampfung 200 angeordnet ist, auf unter 70 °C abgekühlt und von dort aus als gekühltes Produkt-EDC 222 zur Bevorratung geleitet. Alternativ wäre auch eine Anordnung des EDC-Kühlers im Umpump der Natronlauge möglich.

Fig. 3 und Fig. 4 zeigen, wie flüssiges EDC in der Natronlaugeeindampfung 200 verwendet werden kann. In Fig. 3 wird der EDC-Strom 425 aufgezweigt und zum einen Teil 223 in den oberen Abschnitt 201 des Rohrbündels der Natronlaugeeindampfung 200 und zum anderen Teil 224 in einen Einsteckwärmetauscher 211, der als Sumpfbeheizung dient, geführt. Die auf ca. 65-70 °C abgekühlten EDC-Ströme 225 und 226 werden mit dem EDC-Kondensat 220 zusammengeführt und bilden den Rückführstrom 227. In Fig. 4 wird der EDC-Strom 425 nicht aufgezweigt, sondern direkt in den oberen Abschnitt 201 des Rohrbündels der Natronlaugeeindampfung 200 gegeben. Alternativ wäre auch eine Anordnung im Umpump der Natronlauge möglich. Der auf ca. 70 °C abgekühlte EDC-Strom 225 wird mit dem EDC-Kondensat 220 zusammengeführt und beide bilden den Rückführstrom 227.

Der Rückführstrom 227 wird aufgeteilt in die EDC-Teilströme 209 und 212. Das weitere, in den Fig. 3 und 4 dargestellte Verfahren entspricht hinsichtlich der Natronlaugeeindampfung und der Direktchlorierung dem bereits in Fig. 1 beschriebenen.

Zur Veranschaulichung der in Fig. 3 und 4 dargestellten Verfahrensvarianten dient das folgende Zahlenbeispiel auf der Basis einer Simulationsrechnung: Zugrunde gelegt wird dabei eine Anlage mit einer Kapazität von 250 000 Jahrestonnen EDC. Bei einer Anlage dieser Größe beträgt die Reaktions-enthalpie ca. 19,1 MW (218 kJ/mol EDC). Davon können zurückgewonnen werden:

| | |
|---|---|
| Durch Kolonnenbeheizung mit EDC-Brüden: | 7900 kW |
| Durch Kolonnenbeheizung mit Flüssig-EDC: | 2050 kW |
| Durch Feedvorwärmung mit Flüssig-EDC: | 1310 kW |
| Summe: | 11260 kW |

Dies sind ca. 60 % der gesamten Reaktionswärme.

Einer Kapazität von 250 000 Jahrestonnen EDC entspricht ein Chlorbedarf von 22,5 t/h, was wiederum einer Natronlaugeproduktion (als 100-%ig gerechnet) von ca. 25,4 Uh entspricht. Die Natronlauge fällt mit einer Konzentration von 33 % bei einer Temperatur von ca. 80°C an und wird durch Vakuumeindampfung auf 50 % aufkonzentriert. Dies entspricht einer zu verdampfenden Wassermenge von ca. 26,2 t/h bzw. einer Wärmeleistung von 14,6 MW. Hiervon können durch Abkühlung des Kreislauf-EDC-Stroms von 100 °C auf 70 °C in einem Natronlauge-Verdampfer zusätzlich ca. 4,2 MW zurückgewonnen werden. Der Grad der Reaktionswärmeausnutzung verbessert sich damit von 60 % auf 80 %: Die restliche abzuführende Reaktionswärme wird durch Wärmetauscher in der Direktchlorierungsanlage abgeführt.

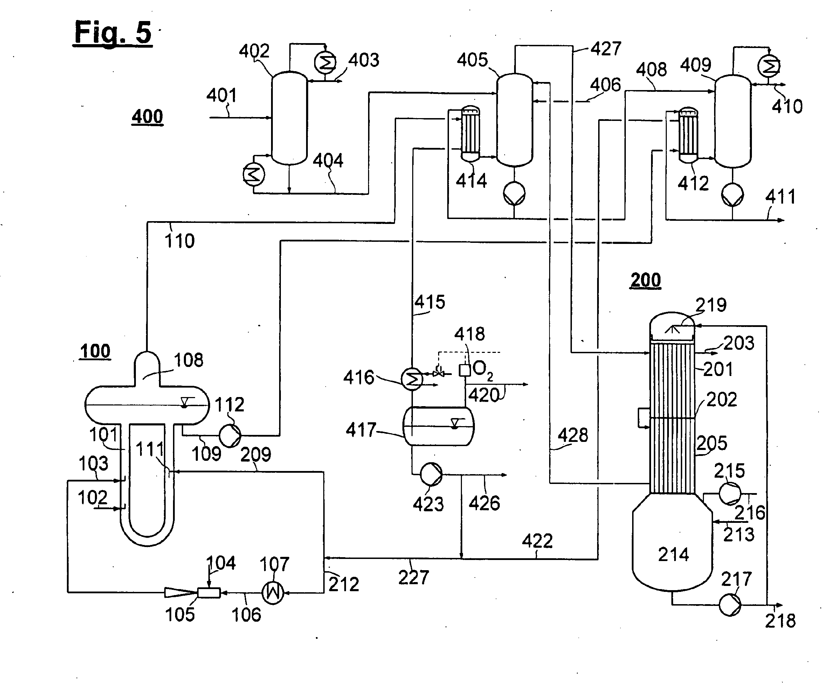

Fig. 5 zeigt ebenso wie Fig. 3 und 4 das ausgewogene VCM-Verfahren, bei dem die Reaktionswärme der Direktchlorierung 100 für die Beheizung von Reinigungskolonnen eingesetzt wird. Unabhängig davon, wie die weitere Wärmenutzung der die Aufkocher verlassenden EDC-Ströme erfolgt, also etwa im Sinne der Erfindung, wie sie in Fig. 3 und 4 dargestellt wird oder auch anderweitig, kann auch der Brüden der Hochsiederkolonne 405 noch zur Natronlaugeeindampfung genutzt werden. Hierzu wird der Hochsiederbrüden 427 statt in einen konventionellen Kondensator in den oberen Abschnitt 201 des Rohrbündels der Natronlaugeeindampfung 200 eingespeist, und das der Natronlaugeeindampfung entnommene EDC-Kondensat wird als Rückfluss 428 in die Hochsiederkolonne 405 zurückgeführt und dort auf den Kolonnenkopf aufgeben.

Beim Hochsiederbrüden 427 handelt es sich um Rein-EDC, mit welchem auf analoge Weise wie mit dem gasförmigen EDC 110, wie dargestellt in Fig. 1, oder wie mit dem EDC-Brüden 303, wie dargestellt in Fig. 2, verfahren werden kann, auf die jeweiligen Beschreibungen sei hier verwiesen. Da es sich um Rein-EDC handelt, ist auch die Vermischbarkeit mit anderen EDC-Brüden oder EDC-Kondensaten des Verfahrens, die ähnliche Reinheit aufweisen, möglich. Daher sind die in Fig. 3, 4 und 5 dargestellten Verfahren gut miteinander kombinierbar.

Zur Veranschaulichung dient das folgende Zahlenbeispiel auf der Basis einer Simulationsrechnung: Zugrunde gelegt wird dabei eine Anlage mit einer Kapazität von 400 000 Jahrestonnen EDC. Bei einer Anlage dieser Größe lassen sich in der Hochsiederkolonne bei einem Kopfdruck von 1,11 bar und einer Temperatur von ca. 87 °C ca. 16,2 MW thermische Leistung gewinnen, womit sich ca. 44 t/h Natronlauge (als 100 % gerechnet) von 33 auf 50 Gewichtsprozent aufkonzentrieren lassen.

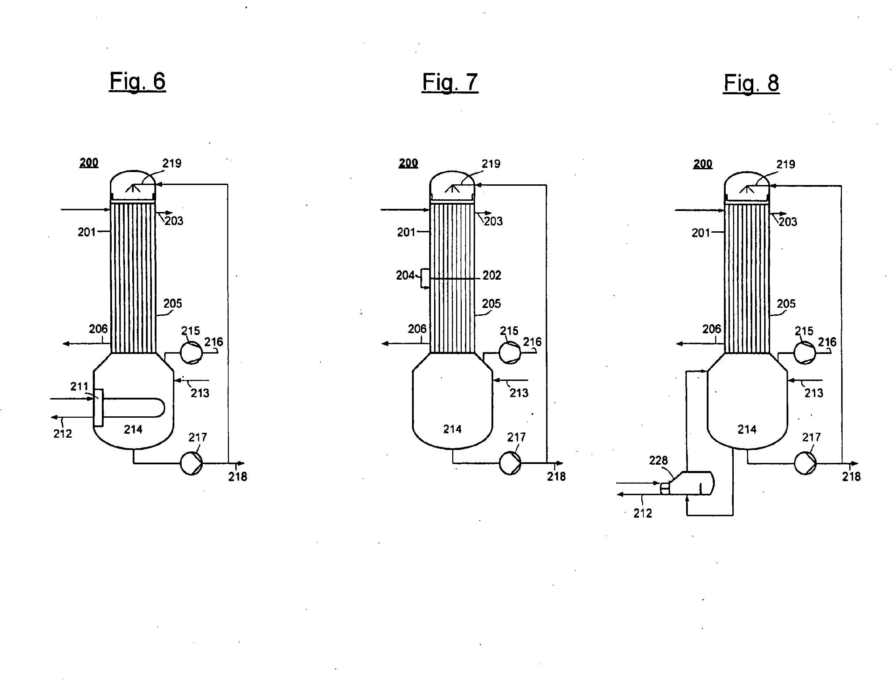

Die Fig. 6 bis 8 zeigen beispielhafte Ausführungsformen der Vorrichtung. Fig. 6 zeigt einen Fallfilmverdampfer ohne horizontale Teilung, bestehend aus einem Rohrbündelwärmetauscher mit 2 festen Rohrplatten und einem NaOH-Sumpfteil, der so ausgeprägt ist, dass Natronlauge rohrinnenseitig und 1,2-Dichlorethan auf der Au-ßenseite der Rohre zu führen ist sowie ferner Einrichtungen aufweist, Natronlauge auf das Rohrinnere aufzugeben und aufzuteilen, sowie Einrichtungen, die auf der Rohrau-ßenseite die Kondensation von 1,2-Dichlorethan ermöglichen und ferner die Zuführung von dampfförmigem 1,2-Dichlorethan sowie die Ableitung von Inertgas und die Ableitung von 1,2-Dichlorethan-Kondensat.

Fig. 7 zeigt außerdem ein Rohrbündel mit Einrichtungen, die die Zuführung von flüssigem 1,2-Dichlorethan sowie dessen Ableitung ermöglichen, wobei ein geteiltes Rohrbündel eingesetzt wird.

Fig. 8 zeigt einen externen Umlaufverdampfer vom Kettle-Typ 228 für den Betrieb mit flüssigem 1,2-Dichlorethan als Heizmedium für den Sumpfteil des Rohrbündelwärmetauschers. Diese Ausführungsform kann vorteilhaft zur Anwendung kommen, wenn mehrere flüssige EDC-Ströme eingesetzt werden, die entweder unterschiedliche Reinheiten aufweisen oder von denen eine Katalysator für den Betrieb des Direktchlorierungsreaktors enthält, und die nicht miteinander vermischt werden dürfen.

### Liste der verwendeten Bezugszeichen

- **100**: **Direktchlorierung**
- 101: flüssigkeitsgefüllte Schlaufe
- 102: Einspeisung von Ethylen
- 103: gelöstes Chlor
- 104: Chlorgas
- 105: Injektor
- 106: flüssiges EDC
- 107: EDC-Kühler
- 108: Ausgasgefäß
- 109: flüssiges EDC
- 110: gasförmiges EDC
- 111: Umlauf-EDC
- 112: Kreislaufpumpe

- **200**: **Natronlaugeeindampfung**
- 201: oberer Mantelraum
- 202: Rohrbündelwärmetauscher
- 203: Inertgasabzug
- 204: EDC-Kondensat
- 205: unterer Mantelraum
- 206: Rein-EDC
- 207: Kondensat-Pumpe
- 208: Produkt-EDC
- 209: Umlauf-EDC
- 210: EDC-Pumpe
- 211: Einsteckkühler
- 212: EDC
- 213: 33-%ige Natronlauge
- 214: Sumpfteil
- 215: Vakuumpumpe
- 216: Wasserdampf
- 217: Natronlaugepumpe
- 218: Produkt-NaOH
- 219: Natronlauge-Verteiler
- 220: EDC-Kondensat
- 221: Abgas
- 222: gekühltes Produkt-EDC
- 223: Teil
- 224: Teil
- 225: EDC-Strom
- 226: EDC-Strom
- 227: Rückführstrom
- 228: Kettle-type Umlaufverdampfer

- **300**: **Destillation**
- 301: Reinigungskolonne
- 302: Brüden
- 303: Brüdenkondensat
- 304: Kopfvorlage
- 305: Vorlagepumpe

- **400**: **EDC-Reinigung**
- 401: Roh-EDC
- 402: Leichtsiederkolonne
- 403: Leichtsiederleitung
- 404: EDC-Leitung
- 405: Hochsiederkolonne
- 406: EDC-Leitung
- 407: Brüdenleitung
- 408: Sumpfstrom
- 409: Vakuumkolonne
- 410: EDC-Brüdenleitung
- 411: Sumpfabzug
- 412: Fallfilmverdampfer
- 413: EDC-Brüden
- 414: Fallfilmverdampfer
- 415: Austrittstrom
- 416: Trimmkondensator
- 417: Vorlage
- 418: Sauerstoffmessgerät
- 419: EDC-Teilstrom
- 420: nichtkondensierbare Anteile
- 421: EDC-Dampfstrom
- 422: EDC-Strom
- 423: Pumpe
- 424: Rein-EDC
- 425: Rein-EDC
- 426: Produkt-EDC
- 427: Hochsiederbrüden
- 428: Rückfluss

## Patentansprüche

1. Verfahren zur Nutzung der Reaktionswärme bei der Herstellung von 1,2-Dichlorethan aus Ethen und Chlor in einem Direktchlorierungsreaktor, wobei das Chlor in einer Natriumchlorid-Elektrolyse erzeugt wird, **dadurch gekennzeichnet, dass** die Reaktionswärme der Bildung von 1,2-Dichlorethan zumindest teilweise für die Eindampfung von NaOH, welches bei der NaCl-Elektrolyse bei der Herstellung des für die Direktchlorierung benötigten Chlors als Koppelprodukt erzeugt wird, genutzt wird.

2. Verfahren nach Anspruch 1, **dadurch gekennzeichnet, dass** die Kondensationswärme des aus der Direktchlorierung abgezogenen 1,2-Dichlorethan-Dampfes zumindest teilweise für die Eindampfung der erzeugten Natronlauge eingesetzt wird.

3. Verfahren nach Anspruch 1, **dadurch gekennzeichnet, dass** die fühlbare Wärme des aus der Direktchlorierung abgezogenen flüssigen 1,2-Dichlorethans zumindest teilweise für die Eindampfung der erzeugten Natronlauge eingesetzt wird.

4. Verfahren nach Anspruch 1, **dadurch gekennzeichnet, dass** die Kondensationswärme der Brüden, die bei der destillativen Reinigung von in einem Direktchlorierungsreaktor aus Ethen und Chlor erzeugtem 1,2-Dichlorethan anfallen, zumindest teilweise für die Eindampfung der erzeugten Natronlauge eingesetzt wird.

5. Verfahren nach Anspruch 1, **dadurch gekennzeichnet, dass** das erzeugte 1,2-Dichlorethan, welches aus dem Reaktor der Direktchlorierung dampfförmig oder flüssig abgezogen wird, zunächst zur indirekten Beheizung von Reinigungskolonnen genutzt, und erst nachdem es einen Teil seiner Wärmeenergie auf relativ hohem Temperaturniveau dort abgegeben hat, zur weiteren Energieabgabe in die Natronlaugeeindampfung weitergegeben wird, wo es Wärmeenergie auf geringerem Temperaturniveau im indirekten Wärmetausch an Natronlauge abgibt.

6. Verfahren nach Anspruch 5, **dadurch gekennzeichnet, dass** die Kondensationswärme der Brüden aus Destillation, deren Aufkocher mit Reaktionswärme betrieben wird, welche bei der Direktchlorierung aus Ethen und Chlor erzeugt wurde, zumindest teilweise für die Eindampfung der erzeugten Natronlauge eingesetzt wird.

7. Verfahren nach Anspruch 5, **dadurch gekennzeichnet, dass** die 1,2-Dichlorethan-enthaltenden Brüden einer Destillationskolonne zur Entfernung von höher als 1,2-Dichlorethan siedenden Komponenten für die Eindampfung von Natronlauge verwendet werden.

8. Vorrichtung zur Durchführung des Verfahrens nach einem der Ansprüche 1 bis 7, bestehend aus einem Rohrbündelwärmetauscher mit 2 festen Rohrplatten und einem NaOH-Sumpfteil, der so ausgeprägt ist, dass Natronlauge rohrinnenseitig und 1,2-Dichlorethan auf der Außenseite der Rohre zu führen ist sowie ferner Einrichtungen aufweist, Natronlauge auf das Rohrinnere aufzugeben und aufzuteilen, sowie einen Anschluss für eine Vakuumpumpe (215) zur Abfuhr von Wasserdampf (216) aufweist.

9. Vorrichtung nach Anspruch 8, aufweisend ein Rohrbündel mit Einrichtungen, die auf der Rohraußenseite die Kondensation von 1,2-Dichlorethan ermöglichen und ferner die Zuführung von dampfförmigem 1,2-Dichlorethan sowie die Ableitung von Inertgas und die Ableitung von 1,2-Dichlorethan-Kondensat.

10. Vorrichtung nach Anspruch 8, aufweisend ein Rohrbündel mit Einrichtungen, die die Zuführung von flüssigem 1,2-Dichlorethan sowie dessen Ableitung ermöglichen.

11. Vorrichtung nach den Ansprüchen 9 und 10, aufweisend ein geteiltes Rohrbündel, dessen einer Teil entsprechend Anspruch 9 und dessen anderer Teil entsprechend Anspruch 10 ausgebildet ist.

12. Vorrichtung nach einem der Ansprüche 8 bis 11, aufweisend einen Einsteckwärmetauscher für den Betrieb mit flüssigem 1,2-Dichlorethan als Heizmedium im Sumpfteil des Rohrbündelwärmetauschers.

13. Vorrichtung nach einem der Ansprüche 8 bis 11, aufweisend einen externen Umlaufverdampfer für den Betrieb mit flüssigem 1,2-Dichlorethan als Heizmedium im Sumpfteil des Rohrbündelwärmetauschers.

## Claims

1. Method for utilising the heat of reaction in the preparation of 1,2-dichloroethane from ethene and chlorine in a direct chlorination reactor, with the chlorine being produced in a sodium chloride electrolysis, **characterised in that** at least part of the heat of reaction from the production of 1,2-dichloroethane is used for the evaporation of NaOH produced as co-product in the NaCl electrolysis for the preparation of the chlorine required for the direct chlorination.

2. Method according to Claim 1, **characterised in that** at least part of the heat of condensation of the 1,2-dichloroethane vapour taken off from the direct chlorination is used for the evaporation of the sodium hydroxide produced.

3. Method according to Claim 1, **characterised in that** at least part of the sensible heat of the liquid 1,2-dichloroethane taken off from the direct chlorination is used for the evaporation of the sodium hydroxide produced.

4. Method according to Claim 1, **characterised in that** at least part of the heat of condensation of the vapours obtained in the purification by distillation of 1,2-dichloroethane produced from ethene and chlorine in a direct chlorination reactor is used for the evaporation of the sodium hydroxide produced.

5. Method according to Claim 1, **characterised in that** the 1,2-dichloroethane produced, which is taken off in gaseous or liquid form from the reactor for the direct chlorination, is firstly used for the indirect heating of purification columns and only after the EDC has transferred part of its heat energy there at a relatively high temperature is it passed on for further energy discharge to sodium hydroxide evaporation where it transfers heat energy at a lower temperature to sodium hydroxide in indirect heat exchange.

6. Method according to Claim 5, **characterised in that** at least part of the heat of condensation of the vapours from distillation the boiler of which is operated using heat of reaction generated in the direct chlorination of ethene and chlorine is used for the evaporation of the sodium hydroxide produced.

7. Method according to Claim 5, **characterised in that** the 1,2-dichloroethane-containing vapours from a distillation column for removing components which have a boiling point higher than that of 1,2-dichloroethane are used for the evaporation of sodium hydroxide.

8. Apparatus for carrying out the method according to any of Claims 1 to 7, consisting of a shell-and-tube heat exchanger which has two fixed tube plates and an NaOH bottom part and is configured so that sodium hydroxide solution is to be conveyed in the inside of the tubes and 1,2-dichloroethane is to be conveyed on the outside of the tubes and also has facilities for supplying and distributing sodium hydroxide solution into the interior of the tubes.

9. Apparatus according to Claim 8, being equipped with a shell-and-tube apparatus having facilities which make it possible to condense 1,2-dichloroethane on the outside of the tubes and also allow the supply of gaseous 1,2-dichloroethane and the discharge of inert gas and of 1,2-dichloroethane condensate.

10. Apparatus according to Claim 8, being equipped with a shell-and-tube apparatus having facilities which make it possible to supply liquid 1,2-dichloroethane and also to discharge it.

11. Apparatus according to Claims 9 and 10, being equipped with a divided shell-and-tube apparatus one part of which is configured according to Claim 9 and the other part of which is configured according to Claim 10.

12. Apparatus according to any of Claims 8 to 11, being equipped with a plug-in heat exchanger for operation using liquid 1,2-dichloroethane as heating medium in the bottom part of the shell-and-tube heat exchanger.

13. Apparatus according to any of Claims 8 to 11, being equipped with an external circulating evaporator for operation using liquid 1,2-dichloroethane as heating medium in the bottom part of the shell-and-tube heat exchanger.

## Revendications

1. Procédé d'utilisation de la chaleur de réaction lors de la production de 1,2-dichloroéthane à partir d'éthylène et de chlore dans un réacteur de chloration directe, le chlore étant fabriqué par électrolyse de chlorure de sodium, **caractérisé en ce que** la chaleur de réaction provenant de la production de 1,2-dichloroéthane est au moins partiellement utilisée pour l'évaporation du NaOH, coproduit fabriqué lors de l'électrolyse de NaCl lors de la fabrication du chlore nécessaire à la chloration directe.

2. Procédé conformément à la revendication 1, **caractérisé en ce que** la chaleur de condensation de la vapeur de 1,2-dichloroéthane évacuée lors de la chloration directe est au moins partiellement utilisée pour l'évaporation de la solution d'hydroxyde de sodium produite.

3. Procédé conformément à la revendication 1, **caractérisé en ce que** la chaleur sensible du 1,2-dichloroéthane liquide évacué lors de la chloration directe est au moins partiellement utilisée pour l'évaporation de la solution d'hydroxyde de sodium produite.

4. Procédé conformément à la revendication 1, **caractérisé en ce que** la chaleur de condensation des vapeurs, produite lors de l'épuration par distillation du 1,2-dichloroéthane produit à partir d'éthylène et de chlore dans un réacteur de chloration directe, est au moins partiellement utilisée pour l'évaporation de la solution d'hydroxyde de sodium produite.

5. Procédé conformément à la revendication 1, **caractérisé en ce que** le 1,2-dichloroéthane produit, évacué du réacteur de chloration directe à l'état de vapeur ou de liquide, est d'abord utilisé pour le chauffage indirect des colonnes d'épuration, puis, après y avoir perdu une partie de son énergie thermique à un niveau de température relativement élevé, est transmis à l'évaporation de la solution d'hydroxyde de sodium pour y libérer son énergie thermique à la solution d'hydroxyde de sodium dans un échange thermique indirect à un niveau de température bas.

6. Procédé conformément à la revendication 5, **caractérisé en ce que** la chaleur de condensation des vapeurs provenant de la distillation, dont le brûleur est exploité avec la chaleur de réaction produite lors de la chloration directe à partir d'éthylène et de chlore, est au moins partiellement utilisée pour l'évaporation de la solution d'hydroxyde de sodium produite.

7. Procédé conformément à la revendication 5, **caractérisé en ce que** les vapeurs contenant du 1,2-dichloroéthane d'une colonne de distillation destinée à éliminer les composants présentant un point d'ébullition supérieur au 1,2-dichloroéthane sont utilisées pour l'évaporation de la solution d'hydroxyde de sodium.

8. Dispositif pour réaliser le procédé conformément à une des revendications 1 à 7, composé d'un échangeur thermique à faisceau tubulaire avec 2 platines fixes et un partie basse NaOH, configuré pour transporter la solution d'hydroxyde de sodium sur la face intérieure des tubes et le 1,2-dichloroéthane sur la face extérieure des tubes et présentant de plus, des dispositifs pour alimenter et distribuer la solution d'hydroxyde de sodium dans l'intérieur du tube.

9. Dispositif conformément à la revendication 8, présentant un faisceau tubulaire avec des dispositifs permettant la condensation du 1,2-dichloroéthane sur la face intérieure du tube ainsi que l'alimentation de 1,2-dichloroéthane à l'état de vapeur tout comme l'évacuation de gaz inerte et l'évacuation de condensat de 1,2-dichloroéthane.

10. Dispositif conformément à la revendication 8, présentant un faisceau tubulaire avec des dispositifs permettant l'alimentation de 1,2-dichloroéthane liquide ainsi que son évacuation.

11. Dispositif conformément aux revendications 9 et 10, présentant un faisceau tubulaire subdivisé, dont une partie est configurée conformément à la revendication 9 et l'autre partie conformément à la revendication 10.

12. Dispositif conformément à une des revendications 8 à 11, présentant un échangeur thermique enfichable exploité avec du 1,2-dichloroéthane liquide comme agent chauffant dans la partie basse de l'échangeur thermique à faisceau tubulaire.

13. Dispositif conformément à une des revendications 8 à 11, présentant un évaporateur à recirculation externe, exploité avec du 1,2-dichloroéthane liquide comme agent chauffant dans la partie basse de l'échangeur thermique à faisceau tubulaire.
